# EUROPEAN PATENT APPLICATION

(11) **EP 3 744 737 A1**
(43) Date of publication of application: **02.12.2020**
(21) Application number: 18894287.4
(22) Date of filing: 27.12.2018
(51) Int. Cl.: C07K 16/30, C07K 16/18, A61K 39/395, A61P 35/00

(54) **HUMAN ANTIBODY AND FRAGMENTS THEREOF FOR USE IN THE TREATMENT OF GASTRIC CANCER (GC) AND OTHER TYPES OF TUMOURS EXPRESSING THE MICA PROTEIN (MHC CLASS I CHAIN-RELATED PROTEIN A GENE)**

(30) Priority: 29.12.2017 CL 20173503
(71) Applicant: Universidad De Chile, Santiago (CL)
(72) Inventor: MOLINA SAMPAYO, María Carmen, Santiago (CL); AGUILLÓN GUTIERREZ, Juan Carlos, Santiago (CL); HAGER RIBEIRO, Carolina, Santiago (CL); ZÚÑIGA OLATE, Roberto Aquiles, Santiago (CL); COLLAZO MUÑOZ, Norberto Andrés, Santiago (CL); LEIVA ARAYA, Lorenzo Eugenio, Santiago (CL); GUTIERREZ GONZALEZ, Matías Fernando, Santiago (CL); TENEB LOBOS, Jaime Camilo, Santiago (CL); ROMERO TRUJILLO, Alfonso Enrique, Santiago (CL); JEREZ DONOSO, Bastián Nicolás, Santiago (CL)
(74) Representative: Dennemeyer & Associates S.A.
(86) International application number: PCT/CL2018/050151
(87) International publication number: WO 2019/126895

(57) **Abstract**

The present invention relates to fully human bivalent monoclonal antibody that specifically binds to the α1 subunit of MICA, SEQ ID NO: 1, CHARACTERIZED in that said antibody comprises a heavy chain variable domain of SEQ ID NO: 2 and a light chain variable domain of SEQ ID NO: 3.

SEQUENCE LISTING

<110> UNIVERSIDAD DE CHILE

<120> Anticuerpo humano y fragmentos de este, para ser utilizados en el
tratamiento del cáncer gástrico (CG) y eventualmente otro tipo de tumores
que expresen la proteína MICA

<130> 273146-CL

<160> 17

<170> BiSSAP 1.3.6

<210> 1
<211> 20
<212> PRT
<213> Homo sapiens

<400> 1
Arg Asp Leu Thr Gly Asn Gly Lys Asp Leu Arg Met Thr Leu Ala His
1               5                   10                  15
Ile Lys Asp Gln
            20

<210> 2
<211> 132
<212> PRT
<213> Homo sapiens

<400> 2
Gln Val Gln Leu Gln Glu Ser Gly Pro Gly Leu Val Lys Pro Ser Gln
1               5                   10                  15
Thr Leu Ser Leu Thr Cys Thr Val Ser Gly Asp Ser Val Arg Ser Ala
            20                  25                  30
Tyr Ser Ser Trp Asn Trp Ile Arg Gln Pro Pro Gly Lys Gly Leu Glu
        35                  40                  45
Trp Ile Gly Gln Ile His Pro Asn Gly Asn Thr Tyr Tyr Asn Pro Ser
    50                  55                  60
Leu Met Ser Arg Ile Thr Val Ser Ile Asp Thr Ser Lys Asn Gln Phe
65                  70                  75                  80
Ser Leu Lys Leu Asp Ser Val Thr Ala Ala Asp Thr Ala Val Tyr Phe
                85                  90                  95
Cys Ala Thr Glu Gly Val Thr Ser Glu Ser Gly Ser Phe Asp Ile Trp
            100                 105                 110
Gly Gln Gly Arg Met Val Ile Val Ser Ser Ala Ser Thr Lys Gly Pro
        115                 120                 125
Ser Val Ala Ser
    130

<210> 3
<211> 114
<212> PRT
<213> Homo sapiens

<400> 3
Glu Phe Gly Val Val Met Thr Gln Ser Pro Asp Phe Gln Ser Val Thr
1               5                   10                  15
Pro Lys Glu Lys Val Thr Ile Thr Cys Arg Ala Ser Gln Ser Ile Gly
            20                  25                  30

Arg Ser Ile His Trp Tyr Gln Gln Lys Pro Asp Gln Ser Pro Lys Leu
        35                  40                  45
Leu Val Met Tyr Ala Ser Gln Ser Phe Ser Gly Val Pro Ser Arg Phe
    50                  55                  60
Ser Gly Ser Gly Ser Gly Thr Asp Phe Thr Leu Thr Ile Asn Ser Leu
65                  70                  75                  80
Glu Ala Glu Asp Ala Ala Thr Tyr Tyr Cys His Gln Ser Tyr Ser Val
                85                  90                  95
Pro Arg Ser Phe Gly Gln Gly Thr Lys Leu Glu Ile Lys Arg Thr Val
            100                 105                 110
Ala Ala

<210> 4
<211> 342
<212> DNA
<213> Homo sapiens

<400> 4
gaattcggtg ttgtgatgac tcagtctcca gattttcagt ctgtgactcc aaaggagaag      60

gtcaccatca cctgccgggc cagtcagagc attggtcgta gtatacactg gtaccagcag     120

aaaccagatc agtctccaaa gctcctcgtc atgtatgctt cccagtcctt ctcaggggtc     180

ccctcgaggt tcagtggcag tggatctggg acagatttca ccctcaccat caatagcctg     240

gaagctgaag atgctgcaac gtattactgc catcagagtt atagtgtacc tcggagtttt     300

ggccagggga ccaagctgga gatcaaacga actgtggctg ca                        342

<210> 5
<211> 396
<212> DNA
<213> Homo sapiens

<400> 5
caggtgcagc tgcaggagtc gggcccagga ctggtgaagc cttcacagac cctgtccctc      60

acctgcactg tctctggtga ctccgtcaga agtgcctatt cctcctggaa ctggatccgg     120

cagccaccag ggaagggcct ggagtggatt gggcagattc atcctaatgg gaacacctac     180

tacaacccgt ccctcatgag tcgaatcacc gtgtctatag acacgtccaa gaaccagttt     240

tccctgaagc tggactctgt gaccgccgcg gacacggccg tttatttctg tgccactgag     300

ggtgttacct ccgagtcggg ctcttttgat atctggggcc aaggcagaat ggtcatcgtc     360

tcttcagctt ccaccaaggg cccatcggtc gctagc                               396

<210> 6
<211> 11
<212> PRT
<213> Homo sapiens

<400> 6
Arg Ala Ser Gln Ser Ile Gly Arg Ser Ile His

1                   5                       10

<210> 7
<211> 7
<212> PRT
<213> Homo sapiens


<400> 7
Met Tyr Ala Ser Gln Ser Phe
1                   5

<210> 8
<211> 9
<212> PRT
<213> Homo sapiens


<400> 8
His Gln Ser Tyr Ser Val Pro Arg Ser
1                   5

<210> 9
<211> 13
<212> PRT
<213> Homo sapiens


<400> 9
Gly Asp Ser Val Arg Ser Ala Tyr Ala Ser Ser Trp Asn
1                   5                       10

<210> 10
<211> 16
<212> PRT
<213> Homo sapiens


<400> 10
Gly Ile His Pro Asn Gly Asn Thr Tyr Tyr Asn Pro Ser Leu Met Ser
1                   5                       10                      15



<210> 11
<211> 12
<212> PRT
<213> Homo sapiens


<400> 11
Glu Gly Val Thr Ser Glu Ser Gly Ser Phe Asp Ile
1                   5                       10

<210> 12
<211> 18
<212> PRT
<213> Artificial Sequence


<220>
<223> Linker sequence

<400> 12
Gly Gly Ser Ser Arg Ser Ser Ser Ser Gly Gly Gly Gly Ser Gly Gly
1               5                   10                  15
Gly Gly


<210> 13
<211> 17
<212> PRT
<213> Artificial Sequence


<220>
<223> Linker sequence

<400> 13
Gly Gly Ser Ala Asp Asp Ala Lys Lys Asp Ala Ala Lys Lys Asp Gly
1               5                   10                  15
Gly


<210> 14
<211> 264
<212> PRT
<213> Artificial Sequence
<220> Unsure
<223> Linker sequence


<400> 14
Glu Phe Gly Val Val Met Thr Gln Ser Pro Asp Phe Gln Ser Val Thr
1               5                   10                  15
Pro Lys Glu Lys Val Thr Ile Thr Cys Arg Ala Ser Gln Ser Ile Gly
            20                  25                  30
Arg Ser Ile His Trp Tyr Gln Gln Lys Pro Asp Gln Ser Pro Lys Leu
        35                  40                  45
Leu Val Met Tyr Ala Ser Gln Ser Phe Ser Gly Val Pro Ser Arg Phe
    50                  55                  60
Ser Gly Ser Gly Ser Gly Thr Asp Phe Thr Leu Thr Ile Asn Ser Leu
65                  70                  75                  80
Glu Ala Glu Asp Ala Ala Thr Tyr Tyr Cys His Gln Ser Tyr Ser Val
                85                  90                  95
Pro Arg Ser Phe Gly Gln Gly Thr Lys Leu Glu Ile Lys Arg Thr Val
            100                 105                 110
Ala Ala Gly Gly Ser Ser Arg Ser Ser Ser Ser Gly Gly Gly Gly Ser
        115                 120                 125
Gly Gly Gly Gly Gln Val Gln Leu Gln Glu Ser Gly Pro Gly Leu Val
    130                 135                 140
Lys Pro Ser Gln Thr Leu Ser Leu Thr Cys Thr Val Ser Gly Asp Ser
145                 150                 155                 160
Val Arg Ser Ala Tyr Ser Ser Trp Asn Trp Ile Arg Gln Pro Pro Gly
                165                 170                 175
Lys Gly Leu Glu Trp Ile Gly Gln Ile His Pro Asn Gly Asn Thr Tyr
            180                 185                 190
Tyr Asn Pro Ser Leu Met Ser Arg Ile Thr Val Ser Ile Asp Thr Ser
        195                 200                 205
Lys Asn Gln Phe Ser Leu Lys Leu Asp Ser Val Thr Ala Ala Asp Thr
    210                 215                 220
Ala Val Tyr Phe Cys Ala Thr Glu Gly Val Thr Ser Glu Ser Gly Ser
225                 230                 235                 240
Phe Asp Ile Trp Gly Gln Gly Arg Met Val Ile Val Ser Ser Ala Ser
                245                 250                 255
Thr Lys Gly Pro Ser Val Ala Ser

            260

<210> 15
<211> 496
<212> PRT
<213> Artificial Sequence
<220> Unsure
<223> Linker sequence

<400> 15
Glu Phe Gly Val Val Met Thr Gln Ser Pro Asp Phe Gln Ser Val Thr
1               5                   10                  15
Pro Lys Glu Lys Val Thr Ile Thr Cys Arg Ala Ser Gln Ser Ile Gly
            20                  25                  30
Arg Ser Ile His Trp Tyr Gln Gln Lys Pro Asp Gln Ser Pro Lys Leu
        35                  40                  45
Leu Val Met Tyr Ala Ser Gln Ser Phe Ser Gly Val Pro Ser Arg Phe
    50                  55                  60
Ser Gly Ser Gly Ser Gly Thr Asp Phe Thr Leu Thr Ile Asn Ser Leu
65                  70                  75                  80
Glu Ala Glu Asp Ala Ala Thr Tyr Tyr Cys His Gln Ser Tyr Ser Val
                85                  90                  95
Pro Arg Ser Phe Gly Gln Gly Thr Lys Leu Glu Ile Lys Arg Thr Val
            100                 105                 110
Ala Ala Gly Gly Ser Ser Arg Ser Ser Ser Ser Gly Gly Gly Gly Ser
        115                 120                 125
Gly Gly Gly Gly Gln Val Gln Leu Gln Glu Ser Gly Pro Gly Leu Val
    130                 135                 140
Lys Pro Ser Gln Thr Leu Ser Leu Thr Cys Thr Val Ser Gly Asp Ser
145                 150                 155                 160
Val Arg Ser Ala Tyr Ser Ser Trp Asn Trp Ile Arg Gln Pro Pro Gly
                165                 170                 175
Lys Gly Leu Glu Trp Ile Gly Gln Ile His Pro Asn Gly Asn Thr Tyr
            180                 185                 190
Tyr Asn Pro Ser Leu Met Ser Arg Ile Thr Val Ser Ile Asp Thr Ser
        195                 200                 205
Lys Asn Gln Phe Ser Leu Lys Leu Asp Ser Val Thr Ala Ala Asp Thr
    210                 215                 220
Ala Val Tyr Phe Cys Ala Thr Glu Gly Val Thr Ser Glu Ser Gly Ser
225                 230                 235                 240
Phe Asp Ile Trp Gly Gln Gly Arg Met Val Ile Val Ser Ser Ala Ser
                245                 250                 255
Thr Lys Gly Pro Ser Val Ala Ser Glu Pro Lys Ser Cys Asp Lys Thr
            260                 265                 270
His Thr Cys Pro Pro Cys Pro Ala Pro Glu Leu Leu Gly Gly Pro Ser
        275                 280                 285
Val Phe Leu Phe Pro Pro Lys Pro Lys Asp Thr Leu Met Ile Ser Arg
    290                 295                 300
Thr Pro Glu Val Thr Cys Val Val Val Asp Val Ser His Glu Asp Pro
305                 310                 315                 320
Glu Val Lys Phe Asn Trp Tyr Val Asp Gly Val Glu Val His Asn Ala
                325                 330                 335
Lys Thr Lys Pro Arg Glu Glu Gln Tyr Asn Ser Thr Tyr Arg Val Val
            340                 345                 350
Ser Val Leu Thr Val Leu His Gln Asp Trp Leu Asn Gly Lys Glu Tyr
        355                 360                 365
Lys Cys Lys Val Ser Asn Lys Ala Leu Pro Ala Pro Ile Glu Lys Thr
    370                 375                 380
Ile Ser Lys Ala Lys Gly Gln Pro Arg Glu Pro Gln Val Tyr Thr Leu
385                 390                 395                 400
Pro Pro Ser Arg Asp Glu Leu Thr Lys Asn Gln Val Ser Leu Thr Cys
                405                 410                 415

Leu Val Lys Gly Phe Tyr Pro Ser Asp Ile Ala Val Glu Trp Glu Ser
            420                 425                 430
Asn Gly Gln Pro Glu Asn Asn Tyr Lys Thr Thr Pro Pro Val Leu Asp
        435                 440                 445
Ser Asp Gly Ser Phe Phe Leu Tyr Ser Lys Leu Thr Val Asp Lys Ser
    450                 455                 460
Arg Trp Gln Gln Gly Asn Val Phe Ser Cys Ser Val Met His Glu Ala
465                 470                 475                 480
Leu His Asn His Tyr Thr Gln Lys Ser Leu Ser Leu Ser Pro Gly Lys
                485                 490                 495

<210> 16
<211> 216
<212> PRT
<213> Artificial Sequence
<220> Unsure
<223> Linker sequence

<400> 16
Glu Phe Gly Val Val Met Thr Gln Ser Pro Asp Phe Gln Ser Val Thr
1               5                   10                  15
Pro Lys Glu Lys Val Thr Ile Thr Cys Arg Ala Ser Gln Ser Ile Gly
            20                  25                  30
Arg Ser Ile His Trp Tyr Gln Gln Lys Pro Asp Gln Ser Pro Lys Leu
        35                  40                  45
Leu Val Met Tyr Ala Ser Gln Ser Phe Ser Gly Val Pro Ser Arg Phe
    50                  55                  60
Ser Gly Ser Gly Ser Gly Thr Asp Phe Thr Leu Thr Ile Asn Ser Leu
65                  70                  75                  80
Glu Ala Glu Asp Ala Ala Thr Tyr Tyr Cys His Gln Ser Tyr Ser Val
                85                  90                  95
Pro Arg Ser Phe Gly Gln Gly Thr Lys Leu Glu Ile Lys Arg Thr Val
            100                 105                 110
Ala Ala Ala Ser Val Phe Ile Phe Pro Pro Ser Asp Glu Gln Leu Lys
        115                 120                 125
Ser Gly Thr Ala Ser Val Val Cys Leu Leu Asn Asn Phe Tyr Pro Arg
    130                 135                 140
Glu Ala Lys Val Gln Trp Lys Val Asp Asn Ala Leu Gln Ser Gly Asn
145                 150                 155                 160
Ser Gln Glu Ser Val Thr Glu Gln Asp Ser Lys Asp Ser Thr Tyr Ser
                165                 170                 175
Leu Ser Ser Thr Leu Thr Leu Ser Lys Ala Asp Tyr Glu Lys His Lys
            180                 185                 190
Val Tyr Ala Cys Glu Val Thr His Gln Gly Leu Ser Ser Pro Val Thr
        195                 200                 205
Lys Ser Phe Asn Arg Gly Glu Cys
    210                 215

<210> 17
<211> 452
<212> PRT
<213> Artificial Sequence
<220> Unsure
<223> Linker sequence

<400> 17
Gln Val Gln Leu Gln Glu Ser Gly Pro Gly Leu Val Lys Pro Ser Gln
1               5                   10                  15
Thr Leu Ser Leu Thr Cys Thr Val Ser Gly Asp Ser Val Arg Ser Ala

20                  25                  30
Tyr Ser Ser Trp Asn Trp Ile Arg Gln Pro Pro Gly Lys Gly Leu Glu
        35                  40                  45
Trp Ile Gly Gln Ile His Pro Asn Gly Asn Thr Tyr Tyr Asn Pro Ser
    50                  55                  60
Leu Met Ser Arg Ile Thr Val Ser Ile Asp Thr Ser Lys Asn Gln Phe
65                  70                  75                  80
Ser Leu Lys Leu Asp Ser Val Thr Ala Ala Asp Thr Ala Val Tyr Phe
                85                  90                  95
Cys Ala Thr Glu Gly Val Thr Ser Glu Ser Gly Ser Phe Asp Ile Trp
            100                 105                 110
Gly Gln Gly Arg Met Val Ile Val Ser Ser Ala Ser Thr Lys Gly Pro
        115                 120                 125
Ser Val Phe Pro Leu Ala Pro Ser Ser Lys Ser Thr Ser Gly Gly Thr
    130                 135                 140
Ala Ala Leu Gly Cys Leu Val Lys Asp Tyr Phe Pro Glu Pro Val Thr
145                 150                 155                 160
Val Ser Trp Asn Ser Gly Ala Leu Thr Ser Gly Val His Thr Phe Pro
                165                 170                 175
Ala Val Leu Gln Ser Ser Gly Leu Tyr Ser Leu Ser Ser Val Val Thr
            180                 185                 190
Val Pro Ser Ser Ser Leu Gly Thr Gln Thr Tyr Ile Cys Asn Val Asn
        195                 200                 205
His Lys Pro Ser Asn Thr Lys Val Asp Lys Lys Val Glu Pro Lys Ser
    210                 215                 220
Cys Asp Lys Thr His Thr Cys Pro Pro Cys Pro Ala Pro Glu Leu Leu
225                 230                 235                 240
Gly Gly Pro Ser Val Phe Leu Phe Pro Pro Lys Pro Lys Asp Thr Leu
                245                 250                 255
Met Ile Ser Arg Thr Pro Glu Val Thr Cys Val Val Val Asp Val Ser
            260                 265                 270
His Glu Asp Pro Glu Val Lys Phe Asn Trp Tyr Val Asp Gly Val Glu
        275                 280                 285
Val His Asn Ala Lys Thr Lys Pro Arg Glu Glu Gln Tyr Asn Ser Thr
    290                 295                 300
Tyr Arg Val Val Ser Val Leu Thr Val Leu His Gln Asp Trp Leu Asn
305                 310                 315                 320
Gly Lys Glu Tyr Lys Cys Lys Val Ser Asn Lys Ala Leu Pro Ala Pro
                325                 330                 335
Ile Glu Lys Thr Ile Ser Lys Ala Lys Gly Gln Pro Arg Glu Pro Gln
            340                 345                 350
Val Tyr Thr Leu Pro Pro Ser Arg Asp Glu Leu Thr Lys Asn Gln Val
        355                 360                 365
Ser Leu Thr Cys Leu Val Lys Gly Phe Tyr Pro Ser Asp Ile Ala Val
    370                 375                 380
Glu Trp Glu Ser Asn Gly Gln Pro Glu Asn Asn Tyr Lys Thr Thr Pro
385                 390                 395                 400
Pro Val Leu Asp Ser Asp Gly Ser Phe Phe Leu Tyr Ser Lys Leu Thr
                405                 410                 415
Val Asp Lys Ser Arg Trp Gln Gln Gly Asn Val Phe Ser Cys Ser Val
            420                 425                 430
Met His Glu Ala Leu His Asn His Tyr Thr Gln Lys Ser Leu Ser Leu
        435                 440                 445
Ser Pro Gly Lys
    450

## Description

### Field of the invention

The developed invention corresponds to a fully human bivalent anti-MICA antibody, heterodimer formed by two variable domains contained in separate peptide chains, a heavy chain and a light chain, and/or homodimer, formed by two variable domains contained in a single peptide chain linked by a linker peptide (scFv), linked to the CH2 and CH3 domains of immunoglobulin. These monoclonal antibodies were designed to specifically bind to the α1 subunit of MICA. Functionally it is capable of binding to the MICA protein in its soluble state, and is capable of binding to tumour cells that express the MICA antigen, thereby activating the effector functions of the antibodies, allowing the tumour cells that express the MICA antigen to be opsonized, activating the system complement and inducing its cellular cytolysis through CD16 (ADCC) or facilitating apoptotic tumor cell phagocytosis by dendritic cells. Additionally, the inactivation of the antigen by one of the two mechanisms reverses the inactivation of NK cells against these tumors, stimulating antitumor immunity.

### Background

The present invention consists of a monoclonal antibody that binds to the MICA protein for the treatment of diseases in which it is desirable to regulate the expression of this protein, such as gastric cancer or other cancers that express it in soluble form or in high concentrations on the surface of tumor cells. The invention is based on new monoclonal antibodies directed against the α1 subunit of MICA, in its soluble state as well as in its membrane-bound state of tumor cells. It specifically binds to the α1 subunit of MICA peptide SEQ ID NO: 1. These antibodies are defined by their heavy and light chain variable strands (or variable domains), which are described in the present application. The invention also comprises pharmaceutical compositions containing the monoclonal antibody to be used in the treatment of gastric cancer or other cancers whose tumor cells express MICA in soluble form or abundantly on its surface.

In order to better illustrate the invention, the present document describes the present invention considering some cases in which it can be applied, however, these examples should not be considered as limiting the invention.

Gastric cancer is the leading cause of cancer-related death worldwide, with an average of less than twelve months of survival when cancer is diagnosed in advanced stages. Overexpression of MICA has been observed in the epithelium of patients with this type of cancer. However, this ligand can be released from the surface of transformed cells, and this in its soluble form is capable of binding naturally to the cell activator receptor "killer" or naturally cytolytic (NK), member D of group 2 (NKG2D), a lectin-like type 2 transmembrane receptor . This effect causes said receptors to be saturated with soluble MICA ligand, resulting in them not being able to efficiently recognize the MICA ligand present in tumor cells. Furthermore, the release of MICA from the surface of the tumor cells causes a decrease in the density of these ligands, generating a decrease in the recognition of the tumor cells by the natural "Killer" cells.

Both effects favor tumor progression in this type of cancer. This is why the need to develop agents that block/decrese the activity of MICA in patients who have been diagnosed with gastric cancer has recently been recognized.

There are commercially available immunogenic preparations and monoclonal antibodies that bind to MICA, which are described as useful to inhibit the proliferation of cancer cells. To assess the merits of the invention described in this document, a brief summary of the most relevant documents forming part of the state of art are presented. Among the most relevant documents stands out, WO2015179627A1 which discloses a method to treat or alleviate cancer patients by means of which an antibody directed against the soluble version of the MICA antigen is used. However, the patent application addresses a method without describing the structure of the antibodies developed in said invention. WO2017157895A1 describes antigen binding proteins and antibodies that bind to MICA polypeptides, which are defined by the heavy and light chain variable strands, but the use of these is only claimed to bind to the MICA antigen expressed in target cells and is not indicated for use in binding soluble MICA.

### Brief description of the invention

The invention corresponds to a fully human antibody, consisting of two variable domain heavy chains and constant domains and two variable domain light chains and constant domain, or consisting of two chains with four immunoglobulin domains; one heavy chain variable domain, another light chain domain linked by a single chain (scFv) and the immunoglobulin constant region CH2 and CH3 domains, which specifically bind to the MICA α1 subunit and which are defined at the structure level. Monoclonal antibodies possess a SEQ ID NO: 2 heavy chain variable region and have a SEQ ID NO: 3 light chain variable region. The embodiment of the invention has several possibilities. Among them are nucleic acids, vectors, cells, compositions, methods and uses of the antibodies developed in the present invention to be used in the treatment of cancer that overexpress MICA, including gastric cancer.

### Description of figures

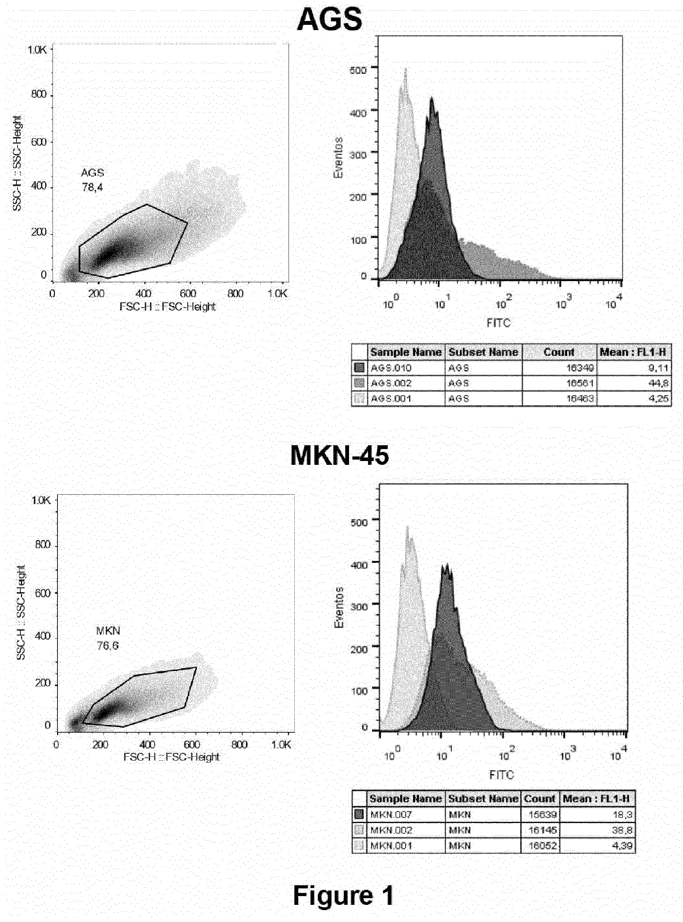

**Figure 1** **. Detection of scFv binding by native MICA.** AGS (A) and MKN-45 (B) gastric cancer lines were incubated with commercial anti MICA antibody or anti MICA scFv (2h, 37 ° C). After washing, the cells were incubated with FITC-conjugated mouse anti-IgG those treated with commercial antibody and anti-murine anti-HA epitope and then FITC-conjugated anti-mouse IgG antibodies treated with commercial and anti-murine anti-HA epitope and then FITC-conjugated anti-mouse IgG antibody. The binding of the anti MICA scFv to native MICA was analyzed by flow cytometry, using the AGS (upper panel) and MKN (lower panel) gastric cancer lines. In the left panels, the population used for the analysis is shown and, on the right side, the FITC signal of unlabeled cells (light gray, solid line), cells labeled with a commercial FITC-conjugated anti-MICA antibody (gray, dotted line), and the scFv signal, detected with a FITC-conjugated anti-HA antibody (black, solid line).
   The average fluorescence in the FL1 -H channel is shown in the Table below.

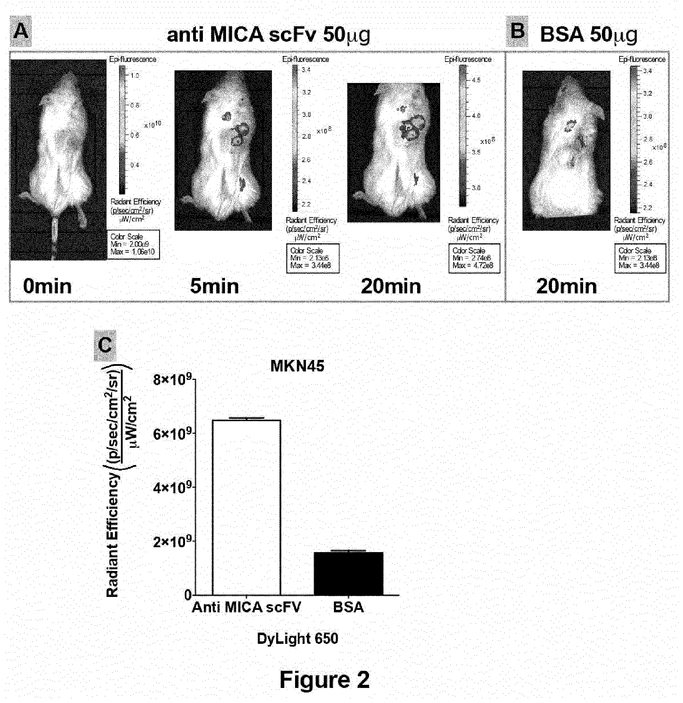

**Figure 2****. Fluorescence emission in NOD-SCID-IL-null (or NSG) mice with MKN-45 cell tumor.** Mice inoculated subcutaneously with 1 x 10 <6> MKN-45 gastric adenocarcinoma cells and once the tumor was established on day 60, 50pg of scFv-MICA conjugated to Dyllght 650 was injected intravenously (lateral tail vein). As a negative control of tumor binding, 50 pg sera bovine albumin (BSA) conjugated to Dylight 650 was used. Mice were intramuscularly anesthetized with a ketamine: xylazine mixture in a 100: 10 v: v ratio, once anesthetized the fluorescence emission in the tumor area was analyzed using IVIS (In Vivo Imagine Systems) LUMINA II. (A) fluorescence emission scFv-MICA-Dyllght 650, 0, 5 and 20 minutes approximately post inoculation. (B) BSA-Dyllght 650 fluorescence emission, approximately 20 minutes post inoculation. (C) Fluorescence emission difference between conjugated BSA and conjugated scFv-MICA approximately 20 minutes post inoculation.

In Figure A, the thoracic region shows two signals; an upper one, which corresponds to the heart and a lower one, which corresponds to the tumor. Both signals are seen at 5 and 20 mln post inoculation, however, it is seen that the signal from the heart is less at 20 minutes while the signal from the tumor is higher, indicating that there is accumulation of scFv-MICA-Dyllght 650 in this latest.

### Detailed description of the invention

The Invention corresponds to fully human bivalent monoclonal antibodies which specifically bind to the MICA α1 subunit, SEQ ID NO: 1, which comprise a heavy chain variable domain SEQ ID NO: 2 and comprise a light chain variable domain of SEQ ID NO: 3.

In a specific preferred embodiment the antibodies are bivalent of a scFv-Fc type chain (where scFv is an antibody fragment consisting of a heavy chain variable domain and a light chain variable domain contained in a heavy chain and a variable domain of light chain contained in a single peptide chain linked through a chorus linker peptide (SEQ ID NO: 12, Linker 1 and SEQ ID NO: 13 Linker 2) formed by the fusion of scFv anti MICA (SEQ ID NO: 14) with CH2 and CH3 domain of human gamma 1 immunoglobulin (SEQ ID NO: 15), and / or bivalent antibody of two Fab-like chains, i.e. the separated peptide chains linked by non-covalent interactions, where the light chain contains CH1 domain of human Kappa immunoglobulin (SEQ ID NO: 16) and the heavy chain containing the CH1, CH2 and CH3 domains of human Gamma 1 immunoglobulin (SEQ ID NO: 17).

In a preferred embodiment of the invention it comprises nucleic acid sequences, which encode for the heavy chain variable domain (SEQ ID NO: 4) and the light or light chain variable domain (SEQ ID NO: 5).

In a more specific embodiment, the antibody or fragments thereof according to the present invention comprises CDR complementary determining regions according to the following definitions: CDR-L1: described in SEQ ID NO: 6; CDR-L2: described in SEQ ID NO: 7; CDR-L3: described in SEQ I D NO: 8; CDR-H1: described in SEQ ID NO: 9; CDR-H2: described in SEQ ID NO: 10; CDR-H3: described in SEQ ID NO: 11.

In a preferred embodiment the invention relates to pharmaceutical compositions comprising monoclonal antibodies, and a pharmaceutically acceptable carrier.

In a preferred embodiment the invention relates to a method of detecting soluble factor MICA in a sample which comprises the following steps: (a) extracting the sample from a patient, from blood or tumor cell culture supernatant (b) placing contact the sample with 0.3 micrograms developed monoclonal antibody, fixed to a micro-titration plate (c) incubate the sample with a secondary anti-MICA antibody conjugated to commercial peroxidase, and (d) quantify the concentration of the soluble factor by means of a signal colorimetric, after adding the enzyme substrate, in a spectrophotometer.

In another preferred embodiment, the invention relates to a kit which comprises a pharmaceutical composition made with the antibody of the invention, which is stored in a pharmaceutically acceptable container.

In another preferred embodiment the invention relates to a method of therapeutic treatment against cancers selected from the following: hepatocellular carcinoma, melanoma, kidney.

In a more particular preferred embodiment the cancer is gastric cancer.

### Examples

**Example 1.- Choice of the epitope or segment of the MICA molecule to which the recombinant antibody will bind to.** In order to select an epitope, and not leave the site of the molecule against which the antibody would bind to random, a Multiple Antigen Peptide System (MAPS) was designed, which consists of a nucleus of lysines and 8 arms of the same peptide. For the design of MAPS, the polymorphisms of the MICA protein, its tertiary structure and its binding site with the NKG2D receptor, obtained from the structure of the NKG2D-MICA complex (PDB: 1 HYR), were analyzed. The least polymorphic MICA alpha helix segment was chosen and the RDLTGNGKDLRMTLAHIKDQ peptide (MICA Alphal) was generated (SEQ ID NO: 1). Subsequently, a massive sequencing analysis of 50 samples from patients with gastric cancer was performed in the laboratory and no variants were found in this segment. Therefore, this segment was selected for the later stages due to its low polymorphic variability.

**Example 2.-** Selection and characterization of a viral particle that expresses anti MICA scFv (scFv-aMICA-phage). Selection or "panning" of the viral particle carrying the gene encoding scFv-aMICA (scFv-aMICA-phage) was performed with the MAPS peptide from a library displaying scFvs on the surface of phage M13, previously constructed in the laboratory at From healthy donors as described in Sotelo et al., (An efficient method for variable region assembly in the construction of scFv phage display libraries using independent strand amplification. mAbs 4, 542-550, doi:10.4161/mabs.20653 (2012)). After 3 cycles of "panning" with the peptide, 70 clones were randomly selected and an ELISA assay was performed, with recombinant MICA-sensitized plates, the peptide and two different proteins blocking the remaining sites of the plate. The clones with the highest signal were selected and their reactivity against recombinant and native MICA (MICAr) was analyzed by ELISA and flow citometry, respectively.

The 5 clones with the highest reactivity against MICA, the scFv-encoding segments were selected, amplified and sequenced. Of these, 3 had the same sequence and a fourth a minor modification, so one of these clones was selected to continue.

**Example 3.-** Generation, production and characterization of the scFv anti MICA protein (scFv-αMICA). To produce the scFv-αMICA protein, the gene encoding it was subcloned into a variant of the pUCH1 phagemid, called pUCH1_Ambar, both built in the laboratory. This variant allows the Independent production of components of the M13 phage capsule and, therefore, its purification. It was observed that the molecule maintains its capacity to bind to MICAr and native protein, respectively, by ELISA assay and in vitro culture citometry of commercial gastric adenocarinoma cell lines (Figure 1).Likewise, the anti-MICA scFv, a monomer molecule,has an affinity constant (KD) of 135 ± 45 nM, Kaff of 7.4 ± 2.5 x 10⁶ M⁻¹. Figure 3 shows a schematic of the model of scFv-aMICA with MICA proposed and subsequently validated in vitro using an animal model of immuno-suppressed mice NOD-SCID IL-2 Rgnull or NSG 45 (NOD.Cg- Prkdcscid II2rgtm1 Wjl / SzJ) (Jackson Laboratories, USA) xenografted with human adenocarcinoma cells (Figure 2).

MICA is a molecule that is absent in mice, thus ensuring the absence of a cross reaction with molecules from the animal. In this model it was verified by immunohistochemistry that human tumor cells, in addition to growing in this murlno model, express MICA. Subsequently, fluorescent scFv-αMICA was inoculated (Figure 2) and it was observed that the scFv-αMICA antibody fragment was significantly located in the tumor unlike fluorescent BSA, its negative control.

## Claims

1. Fully human bivalent monoclonal antibody that specifically binds to the α1 subunit of MICA, SEQ ID NO: 1, **CHARACTERIZED in that** said antibody comprises a heavy chain variable domain of SEQ ID NO: 2 and a light chain variable domain of SEQ ID NO: 3.

2. Monoclonal antibody according to claim 1, **CHARACTERIZED in that** said antibody is bivalent of a scFv-Fc type chain, where scFv is an antibody fragment formed by a heavy chain variable domain and a light chain variable domain contained in a single peptide chain linked through a linker peptide selected from SEQ ID NO: 12 and SEQ ID NO: 13.

3. Monoclonal antibody according to claim 2, **CHARACTERIZED in that** said bivalent antibodies are formed by the fusion of scFv anti MICA (SEQ ID NO: 14) with CH2 and CH3 domains of human immunoglobulin gamma-1 (SEQ ID NO: 15), and/or bivalent antibody of two Fab-type chains, i.e. separate peptides chains linked by non-covalent interactions, where the light chain contains the CH1 domain of human immunoglobulin kappa (SEQ ID NO: 16) and the heavy chain contains the CH1, CH2 and CH3 domains of human immunoglobulin gamma-1 (SEQ ID NO: 17).

4. Monoclonal antibody according to claim 1, **CHARACTERIZED in that** said antibody comprises nucleic acid sequences, which encode for the heavy chain variable domain (SEQ ID NO: 4) and the light or light chain variable domain (SEQ ID NO: 5).

5. Monoclonal antibody according to claim 1, **CHARACTERIZED in that** said antibody or fragments thereof comprise complementary-determining regions (CDR), according to the following definitions: CDR-L1: described in SEQ ID NO: 6; CDR-L2: described in SEQ ID NO: 7; CDR-L3: described in SEQ ID NO: 8; CDR-H1: described in SEQ ID NO: 9; CDR-H2: described in SEQ ID NO: 10; CDR-H3: described in SEQ ID NO: 11.

6. Pharmaceutical composition **CHARACTERIZED in that** said composition comprises the monoclonal antibodies described in claims 1 to 5, and a pharmaceutically acceptable carrier.

7. Method of detection of soluble factor MICA in a sample **CHARACTERIZED in that** said method comprises the following steps:
a. Obtaining the sample from a patient, blood sample or supernatant from tumour cell culture;
b. Putting the sample in contact with 0.3 micrograms of the developed monoclonal antibody, fixed to a micro-titration plate;
c. Incubating the sample with a commercial peroxidase-conjugated anti-MICA secondary antibody, and
d. Quantify the concentration of the soluble factor by means of a colorimetric signal, after adding the enzyme substrate, in a spectrophotometer.

8. Kit **CHARACTERIZED in that** it comprises a pharmaceutical composition made with the antibody or fragments thereof defined in claims 1 to 5, which is stored in a pharmaceutically acceptable container.

9. Use of the antibody described in claims 1 to 5, **CHARACTERIZED IN THAT** said antibody serves to prepare a medicine useful in the treatment of cancers selected from the following: hepatocellular, melanoma, renal, and gastric carcinoma.
